Europäisches Patentamt

European Patent Office

Office européen des brevets

(11) Publication number: **0 097 407**
**B1**

(12) **EUROPEAN PATENT SPECIFICATION**

(45) Date of publication of patent specification: **13.05.87**

(51) Int. Cl.⁴: **A 61 K 39/02**

(21) Application number: **83200929.4**

(22) Date of filing: **22.06.83**

(54) **Vaccines against diseases causing bacteria with capsular polysaccharides and process of preparing them.**

(30) Priority: **22.06.82 NL 8202527**

(43) Date of publication of application:
**04.01.84 Bulletin 84/01**

(45) Publication of the grant of the patent:
**13.05.87 Bulletin 87/20**

(84) Designated Contracting States:
**AT BE CH DE FR GB IT LI LU NL SE**

(56) References cited:
**EP-A-0 002 404**
**EP-A-0 017 557**
**GB-A- 916 455**

**CHEMICAL ABSTRACTS, vol. 95, no. 23, 7th December 1981, page 496, no. 201810z, Columbus, Ohio, USA. C. WOOD et al.: "Immunochemical studies of conjugates of isomaltosyl oliogosaccharides to lipid. I. Antigenicity of the glycolipids and the production of specific antibodies in rabbits"**

(73) Proprietor: **Rijksuniversiteit Utrecht**
**Catharijnesingel 59**
**NL-3511 GG Utrecht (NL)**

(72) Inventor: **Snippe, Harm, Dr.**
**Wikkeoord 2**
**Houten (NL)**
Inventor: **Willers, Johannes Marius Nicolaas, Prof., Dr.**
**Julianalaan 35**
**Maartensdijk (NL)**
Inventor: **van Houte, Arend-Jan, Dr.**
**Koningin Emmastraat 4**
**Bunnik (NL)**
Inventor: **Vliegenthart, Johannes Frederik G., Prof., Dr.**
**Van Zijldreef 20**
**Bunnik (NL)**
Inventor: **Kamerling, Johannis Paulus, Dr.**
**Schansbos 24**
**Linschoten (NL)**
Inventor: **van Dam, Jan Evert Gerard, Drs.**
**Havenstraat 6**
**Amsterdam (NL)**

(74) Representative: **Kooy, Leendert Willem et al**
**OCTROOIBUREAU VRIESENDORP & GAADE**
**P.O. Box 266**
**NL-2501 AW The Hague (NL)**

**0 097 407**

(58) References cited:

**CHEMICAL ABSTRACTS, vol. 71, no. 17, 27th October 1969, page 189, no. 79219a, Columbus, Ohio, USA: M. KATZ et al.: "Quantitative studies of the specificity of anti-pneumococcal antibodies, types III and VIII. IV. Binding of labeled hexasaccharides derived from S3 by anti-S3 antibodies and their Fab fragments"**

## Description

The invention relates to vaccines against pathogenic Streptococcus pneumoniae bacteria and to a process for preparing said kind of vaccines.

The preparation of effective vaccines against pathogenic bacteria, which are envelopped by capsular polysaccharides, is a problem.

Said capsular polysaccharides protect the bacteria very effectively against fagocytosis by cells of the patients. For that reason is has been attempted to find vaccines specifically stimulating the production of antibodies against polysaccharides in human beings and animals. Such antibodies should react with the protective capsules, and destruction of the infective bacteria should follow rapidly and safely. However tests with vaccines against the intact capsular polysaccharides of this type of bacteria have proved that this type of vaccines has limited effect. Since these polysaccharides consist of repeating units, it has been tried whether decomposition products in the form of oligosaccharides from said polysaccharides could be more effective. As a matter of fact it was found that oligosaccharide fractions with 2—12 preferably 6—10 monosaccharide units develop a strong antigenic activity if presented on a protein carrier.

GB Patent Specification 915 455 discloses a synthetic antigen consisting of oligosaccharides obtained from Gram-negative bacterial lipopolysaccharides.

However when administered to humans and animals, said type of oligosaccharides often appeared, during further tests to be decomposed in the body before they could develop their antigenic activity or stimulate the production of antibodies against the intact polysaccharides. So according to the invention a possibility was studied to administer the oligosaccharides in a form adequately protected against decomposition in the body of a patient, being either a human being or an animal, and suitable to sufficiently stimulate the production of antibodies. According to the invention such a protection is achieved effectively when the oligosaccharides are anchored in a liposome via a primary lipophilic alkylamine with 14—22 carbon atoms, or via other amines with a strong hydrophobic portion, such as the phosphatidyl ethanolamines. The present vaccines comprise oligosaccharides which are anchored in liposomes via alkylamines.

Vaccines according to the invention against pathogenic Streptococcus pneumoniae bacteria contain oligosaccharides originating from the capsular polysaccharides of said pathogenic bacteria, which are anchored in a liposome via an alkylamine with 14—22 carbon atoms or a phosphatidyl ethanolamine.

It is very surprising that such a vaccine not only provides immunity, but even produces a considerable improved and longer lasting immunity.

By anchoring oligosaccharides, originating from various Streptococcus pneumonia bacteria, in the liposome, a multivalent vaccine against various bacteria can be obtained. In principle it is also possible to use the same oligosaccharides prepared by a chemical route.

The parts of the present vaccines are described herebelow.

To prepare the most active fractions of oligosaccharides, the intact polysaccharides are used as starting material. The latter can be obtained by cultivating the concerned bacterium in a suitable fermentation medium, neutralising the culture with alkaline solution, precipitating the polysaccharides with calcium chloride or another suitable salt, washing with water and dissolving again several times in solutions of acids, bases or salts followed every time by precipitating for instance with ethanol or acetone. In this way a sufficiently pure polysaccharide fraction is finally obtained. Therefrom the desired oligosaccharides can be obtained by partial hydrolysis. The hydrolysis can be effected enzymatically or chemically, in particular by acidic hydrolysis. By heating the resulting polysaccharide fraction in an acid medium for some time, a mixture comprising the desired oligosaccharide fraction is formed. Usually heating in a 1N-acid solution at 100°C for about 30 minutes appeared to be very suitable. Said acidic hydrolysis provides a number of oligosaccharides with 2 — to many saccharide — units. These oligosaccharides can be separated by chromatography, for instance over Sephadex®, so that oligosaccharides of different chain lengths are recovered and thus separated.

The most active fractions appeared to be those with 6 to 8 monosaccharide-units per molecule. Said fractions can be identified by means of thin-layer chromatography, gas-liquid chromatography, mass spectrometry and nuclear magnetic resonance.

The oligosaccharide fractions thus obtained cannot be directly attached to liposomes, but they have first to be converted into the form of a glycolipid. The best way to do this is by reductively aminating them with an alkylamine and a reducing agent, such as $NaCNBH_3$. This reaction is carried out at room temperature for 7—14 days until the major part of the uncoupled oligosaccharide has disappeared. This is checked by thin-layer chromatography. For a reasonable reaction time, preferably a great excess, rather a fivefold excess of amine and reducing agent are applied. As the reaction medium, any solvent can be used that sufficiently dissolves said reactants.

To obtain the desired glycolipids in a pure form, they are dissolved in a hydrophobic solvent, e.g. chloroform, after the reaction mixture was evaporated to dryness, whereafter an equal volume of water is added, the entire mixture is shaken in order to obtain an emulsion, the emulsion intermediate layer is separated by centrifuging and the desired glycolipid is recovered from this intermediate layer. The glycolipids may also be recovered by washing several times a solution of the reaction products and the starting products with a hydrophobic solvent in order to remove the free and not reacted alkylamine, followed by freeze drying the water/methanol solution and dissolving the residue for instance in chloroform/methanol. The not converted oligosaccharides and reducing agents do not dissolve and can be filtered off. The solution contains pure glycolipid.

The liposomes are in principle prepared in the way as described in J. Mol. Biol. *13*, 238 (1965) and Immunology *37*, 503 (1979). They consist of a lipid double layer, usually built up from phospholipids, such as lecithins, and a sterol, usually cholesterol, the polar groups of which limit the double layer. Between the various double layers there are compartments. The above mentioned glycolipids are anchored in the lipid double layer of the liposomes. Therefore suitable amounts of phospholipid (lecithin), sterol and glycolipid are dissolved, subsequently the solution is evaporated to dryness, the residue is treated in a suitable, preferably physiological salt solution and ultrasonically vibrated for a short period of time.

In this way liquid-suspensions are obtained which, when injected into mammals, provide a good and lasting immunity against the bacteria from which the starting polysaccharides originated. When for example mice are injected with a liposome suspension as described above having a degree of charge of about 5 mol. % of glycolipid in an amount of 1 nmol. glycolipid and 7 days later with a lethal dose of the bacterium, from which the carbohydrate part of the glycolipid originated, it appeared that all the mice were protected. However, if the glycolipid anchored in the liposome is replaced by a corresponding amount of the starting polysaccharide, a corresponding amount of oligosaccharide prepared therefrom or uncharged lipsome, an injection of a lethal dose of the said organism 7 days later appeared to kill all the test animals. Furthermore, the immunity obtained appeared to be specific. With immunisation with oligosaccharide-liposomes exclusively originating from *Str. pneumoniae* type III, no immunity was obtained for example against type IX. After said immunisation against type III an injection of a lethal dose of the latter bacterium was as fatal as before.

For the preparation of a definitive vaccine an adjuvant can be added as usual, for example dimethyl-dioctadecylammonium bromide appears to be satisfactory in the present vaccines.

It was very surprising that polysaccharides treated in this way (hydrolyzed, reductively aminated and anchored to liposome) not only provided a clear immunity, but even produced a considerably improved and longer lasting immunity.

The following examples elucidate the invention further without limiting the invention.

### Example I
Preparation of an active oligosaccharide-fraction

After isolation *Streptococcus pneumoniae* type III was cultivated for 4 days on a brain-heart-infusion broth (Difco) comprising 2 g/l glucose and 5 g/l $NaHCO_3$. Thereafter the cells were recovered, neutralized with 2N sodium hydroxide and 5 g/l calcium chloride were added to the cell suspension. A precipitate of impure capsular polysaccharide was formed that was washed twice with about 20 times the amount of distilled water. Said precipitate was filtered through with Whatman 50® filter-paper.

Thereafter said precipitate was dissolved in water for purification, acidified with 8 $cm^3$ per liter 4N HCl and precipitated with 2 parts by volume of 96% ethanol and washed with 75% ethanol. The residue was dissolved in just sufficient distilled water and after adjusting the pH to 10 with sodium hydroxide, precipitated again with ethanol of 96%. After washing with 75% ethanol the residue was again dissolved in just sufficient distilled water, and after addition of 0.4 g/l sodium acetate again precipitated with 96% ethanol. Finally, the rest of the protein were removed by dissolving the latter precipitate in distilled water, shaking the solution with 1/4 volume of a mixture of chloroform : butanol : glacial acetic acid (20 : 4 : 1 by vol.) and separating off the aqueous phase. After precipitating again with ethanol and taking up in water, the product was freezedried. In this way the pure capsular polysaccharides were isolated.

300 mg of said capsular polysaccharides were dissolved in 15 $cm^3$ of distilled water, 15 $cm^3$ of 2 N $N_2SO_4$ was added thereto and the whole was heated at 100°C for 30 minutes. Subsequently it was neutralized with barium hydroxide and the resulting $BaSO_4$-precipitate was centrifugated.

The supernatant was freezedried and dissolved in 10 $cm^3$ of water. The formed oligosaccharides were fractionated over a column of Sephadex G 25® superfine of Pharmacia (130 × 2.2 cm), using distilled water. The eluate fractions were tested for oligosaccharide by detection with a Uvicord S at 206 mm and, using dextran-blue and $K_2Cr_2O_7$ as column markers.

The oligosaccharide-containing fractions were further purified on Bio-Gel P-4®. On thin-layer chomatography plates (with eluant n-butanol : pyridine : water (6 : 5 : 5 by vol.) and the chain length was determined by gas chromatography by Tsai (Anal. Biochem. *36*, 114 (1979). Subsequently these fractions were tested for their capacity to inhibit an antigen-antibody-reaction of the original polysaccharides. The oligosaccharide-fraction which inhibits said reaction most effectively, is that which best competes with the original polysaccharides, having the highest affinity against the antibodies and containing the antigenic determinant. This appeared to be the oligosaccharide-fraction having 6 monosaccharide units. The oligosaccharide-fractions obtained after the Bio-Gel P-4® fractionation, having an oligosaccharide with 6 monosaccharide units as main component were combined thereafter and used for the following tests.

## Example II

30 mg of the oligomer-fraction which was obtained according to example I, was dissolved in 2.0 cm³ of a mixture of THF and water (5:2) and under stirring 36 mg of stearylamine and 5 mg of NaCNBH₃ were added thereto and the pH was adjusted to 8. Subsequently the mixture was stirred at room temperature for 10 days. Thereafter the reaction mixture was subjected to thin-layer chromatography (with eluant chloroform:methanol:water (13::1 by vol.), and it appeared that non converted oligosaccharide material was no longer present. The resulting glycolipids were recovered by evaporating the reaction mixture to dryness under reduced pressure, dissolving the residue in chloroform, adding an equal volume of water, shaking and separating the resulting emulsion intermediate layer by centrifuging. Subsequently said layer was evaporated to dryness.

## Example III

43.8 mg (30 nmol) of the glycolipid obtained according to example II was dissolved in chloroform:methanol (3:1 by vol.). In a round bottom flask of 50 cm³ said solution was combined with a solution of 374.5 μg (510 nmol) of dipalmitoyl-L-α-phosphatidyl-chlorine in chloroform and a solution of 23.2 μg (60 nmol) of cholesterol in chloroform. After carefully evaporating the chloroform at 37°C a thin film was formed on the wall of the round bottom flask, 15 cm³ of a physiological salt solution buffered with phosphate was added, thereafter it was carefully shaken at 61°C for 10 minutes. Subsequently the whole was allowed to stand at room temperature for 1 hour, vibrated ultrasonically with an amplitude of 7 μm for 30 seconds, each time with an intermediate period of 15 seconds. In this way a liposome was anchored therein a glycolipid produced according to example II was obtained. The liposome preparation was allowed to stand at room temperature for 1 hour before immunization of animals.

## Example IV

10 weeks old female mice were injected with increasing amounts of the material produced according to example II, and viz. to an amount corresponding with 30 nmol of glycolipid (anchored in liposome). After 7 days each group was injected i.p. with 25 times the lethal dose of *Str. pneumoniae* type III. From a dose corresponding with 0.5 nmol of glycolipid onward the protection appeared to be effective. All the control animals died, all the animals immunized with more than 0.5 nmol anchored glycolipid survived. Said protection is specific for *Str. pneumoniae* type III, for a later injection of *Str. pneumoniae* type IX appeared to be lethal to these test animals.

## Claims

1. Vaccines against pathogenic *Streptococcus pneumoniae* bacteria, characterized in that said vaccines contain oligosaccharides originating from the capsular polysaccharides of said pathogenic bacteria, which are anchored in a liposome via an alkylamine with 14—22 carbon atoms or a phosphatidyl ethanolamine.

2. Vaccine according to claim 1, characterized in that the oligosaccharides originate from polysaccharides of different pathogenic bacteria.

3. Vaccine according to claims 1 to 2, characterized in that the oligosaccharides contain 2—12, preferably 6—10 monosaccharide units per molecule.

4. Vaccine according to any one of the preceding claims, characterized in that the alkylamine is a lipophilic primary alkylamine with 16—20 carbon atoms.

5. Vaccine according to any one of the preceding claims, characterized in that the liposome is composed of a double layer of a phospholipid and a sterol.

6. Vaccine according to any one of the preceding claims 1—5, characterized in that it also comprises an adjuvant.

7. Vaccine according to claim 6, characterized in that the adjuvant is dimethyl-dioctadecyl ammonium bromide.

8. A process for preparing a vaccine according to any one of the preceding claims, characterized in that the capsular polysaccharides of *Streptococcus pneumoniae* are hydrolyzed to yield oligosaccharides containing the antigenic determinant which oligosaccharides are reductively aminated with an alkylamine, with 14—22 carbon atoms or a phosphatidyl ethanolamine, and the obtained glycolipids are anchored in a liposome.

9. A process according to claim 8, characterized in that the capsular polysaccharides are hydrolyzed by enzymatic or acidic hydrolysis to oligosaccharides with 6—8 monosaccharide units, said oligosaccharides are reductively aminated with a lipophilic primary alkylamine with 16—20 carbon atoms and NaCNBH₃ and the obtained glycolipids are anchored in a liposome using lecithin and a sterol.

## Patentansprüche

1. Impfstoffe gegen pathogene *Streptococcus pneumoniae* Bakterien, dadurch gekennzeichnet, daß die Impfstoffe von dem Hüllpolysacchariden der pathogenen Bakterien stammende Oligosaccharide enthalten, die über ein Alkylamin mit 14 bis 22 Kohlenstoffatomen oder ein Phosphatidyl-Äthanolamin in einem Liposom verankert sind.

2. Impfstoff nach Anspruch 1, dadurch gekennzeichnet, daß die Oligosaccharide von Polysacchariden unterschiedlicher pathogener Bakterien stammen.

3. Impfstoff nach den Ansprüchen 1 oder 2, dadurch gekennzeichnet, daß die Oligosaccharide 2 bis 12, vorzugsweise 6 bis 10 Monosaccharideinheiten pro Molekül enthalten.

4. Impfstoff nach einem der vorangehenden Ansprüche, dadurch gekennzeichnet, daß das Alkylamin ein lipophiles primäres Alkylamin mit 16 bis 20 Kohlenstoffatomen ist.

5. Impfstoff nach einem der vorangehenden Ansprüche, dadurch gekennzeichnet, daß das Liposom aus einer Doppelschicht aus einem Phospholipid und einem Styrol besteht.

6. Impfstoff nach einem der vorangehenden Ansprüche 1 bis 5, dadurch gekennzeichnet, daß er auch ein Adjuvans enthält.

7. Impfstoff nach Anspruch 6, dadurch gekennzeichnet, daß das Adjuvans Dimethyl-dioctadecyl-Ammoniumbromid ist.

8. Verfahren zur Herstellung eines Impfstoffs nach einem der vorangehenden Ansprüche, dadurch gekennzeichnet, daß man die Hüllpolysaccharide von *Streptococcus pneumoniae* zu Oligosacchariden hydrolysiert, die die antigene Determinante enthalten, die Oligosaccharide reduktiv mit einem Alkylamin mit 14 bis 22 Kohlenstoffatomen oder Phosphatidyl-Äthanolamin aminiert und die erhaltenen Glykolipide in einem Liposom verankert.

9. Verfahren nach Anspruch 8, dadurch gekennzeichnet, daß man die Hüllpolysaccharide durch enzymatische oder saure Hydrolyse zu Oligosacchariden mit 6 bis 8 Monosaccharideinheiten hydrolysiert, die Oligosaccharide mit einem lipophilen primären Amin mit 16 bis 20 Kohlenstoffatomen und NaCNBH₃ reduktiv aminiert und die erhaltenen Glykolipide unter Verwendung von Lecithin und einem Sterin in einem Liposom verankert.

**Revendications**

1. Vaccins contre les bactéries *Streptococcus pneumoniae* pathogènes, caractérisés en ce que lesdits vaccins contiennent des oligosaccharides provenant des polysaccharides capsulaires desdites bactéries pathogènes, qui sont fixés dans un liposome par un alkylamine avec 14—22 atomes de carbone ou une phosphatidyl éthanolamine.

2. Vaccin selon la Revendication 1, caractérisé en ce que les oligosaccharides proviennent de polysaccharides de différentes bactéries pathogènes.

3. Vaccin selon les Revendications 1 ou 2, caractérisé en ce que les oligosaccharides contiennent 2—12, de préférence 6—10 unités monosaccharidiques par molécule.

4. Vaccin selon l'une quelconque des revendications précédentes, caractérisé en ce que l'alkylamine est une alkylamine primaire lipophile avec 16—20 atomes de carbone.

5. Vaccin selon l'une quelconque des revendications précédentes, caractérisé en ce que le liposome est composé d'une double couche d'un phospholipide et d'un stérol.

6. Vaccin selon l'une quelconque des revendications 1—5, précédentes, caractérisé en ce qu'il contient également un adjuvant.

7. Vaccin selon la revendication 6, caractérisé en ce que l'adjuvant est le bromure de diméthyl-di-octadécyl-ammonium.

8. Procédé de préparation d'un vaccin selon l'une quelconque des revendications précédentes, caractérisé en ce que les polysaccharides capsulaires de *Streptococcus pneumoniae* sont hydrolysés pour donner des oligosaccharides contenant le déterminant antigénique, ces oligosaccharides étant soumis à une amination réductrice avec une alkylamine, avec 14—22 atomes de carbone ou une phosphatidyl ethanolamine, et les glycolipides obtenus sont fixés dans un liposome.

9. Procédé selon la revendication 8, caractérisé en ce que les polysaccharides capsulaires sont hydrolysés par hydrolyse enzymatique ou acide en oligosaccharides avec 6—8 unités monosaccharidiques, lesdits oligosaccharides sont soumis à une amination réductrice avec une alkylamine primaire lipophile avec 16—20 atomes de carbone et NaCNBH₃ et les glycolipides obtenus sont fixés dans un liposome en utilisant la lécithine et un stérol.